# EUROPÄISCHE PATENTSCHRIFT

(11) **EP 0 817 595 B1**
(45) Veröffentlichungstag und Bekanntmachung des Hinweises auf die Patenterteilung: **12.06.2002**
(21) Anmeldenummer: 96903870.2
(22) Anmeldetag: 07.03.1996
(51) Int. Cl.: A61B 17/22

(54) **KATHETER ZUM ABLÖSEN VON ABNORMALEN ABLAGERUNGEN IN MENSCHLICHEN BLUTGEFÄSSEN**
CATHETER FOR DETACHING ABNORMAL DEPOSITS IN HUMAN BLOOD VESSELS
CATHETER PERMETTANT DE DETACHER DES DEPOTS ANORMAUX SITUES DANS DES VAISSEAUX SANGUINS CHEZ L'HOMME

(30) Priorität: 28.03.1995 CH 87495
(43) Veröffentlichungstag der Anmeldung: 14.01.1998
(73) Patentinhaber: Straub Medical AG, 7323 Wangs (CH)
(72) Erfinder: STRAUB, Immanuel, CH-7323 Wangs (CH)
(74) Vertreter: Patentanwälte Schaad, Balass, Menzl & Partner AG
(86) Internationale Anmeldenummer: CH9600086
(87) Internationale Veröffentlichungsnummer: WO9629942

(56) Entgegenhaltungen:
- EP-A- 0 448 859
- US-A- 3 320 957
- US-A- 4 857 046
- US-A- 4 955 882
- US-A- 5 269 751
- US-A- 5 383 884

## Beschreibung

Die Erfindung bezieht sich auf einen Katheter der im Oberbegriff des Anspruchs 1 genannten Art, welcher auch unter der Bezeichnung Rotationskatheter bekannt ist.

Ein solcher Katheter dient insbesondere zum Behandeln von arteriellen Verschlusserkrankungen durch Abtragen von Stenosen und Zerkleinern von Thromben. Er wird in die Arterie eingeführt und bis zu der verengten Stelle vorgeschoben, die zu behandeln ist. An seinem vorderen oder vorlaufenden Ende ist ein rotierend antreibbares Schneidwerkzeug angeordnet.

Bei einem aus der US-A-5269751 bekannten Katheter gemäss dem Oberbegriff des Anspruchs 1 dient die auch als Förderschraube augebildete flexible gewendelte Antriebswelle als Führungssonde, welche vor dem Katheter in die Arterie eingeführt und der Katheter darauf nachgeführt wird. Wenn diese gewendelte Antriebwelle ohne den schützenden Katheterschlauch in die Arterie eingeführt wird, ist jedoch das Risiko nicht auszuschliessen, dass die Arterie dabei verletzt wird.

Ein weiterer, beispielsweise aus der EP-B1-0 267 539 bekannter Katheter weist als Schneidwerkzeug einen im wesentlichen ellipsenförmigen Schneidfräser auf, dessen Oberfläche mit abrasivem Material versehen ist und der mit einer Drehzahl bis zu 160'000/min angetrieben wird. Der Schneidfräser ist über eine biegsame Antriebswelle mit einem am anderen Ende des Katheters angeordneten Drehantrieb verbunden. Die Antriebswelle läuft in einer als Katheterschlauch dienenden schlauchförmigen Hülle. Durch die Antriebswelle hindurch erstreckt sich ein Führungsdraht, der vor dem Einführen des Katheters in die Arterie eingeführt und bis durch die Verengung hindurch vorgeschoben wird.

Bei diesem bekannten Rotationskatheter werden die durch den Schneidfräser abgetragenen Partikel nicht aus dem Körper entfernt, da sie normalerweise kleiner als die roten Blutzellen mit ca. 7 µm sein sollten. Falls einzelne abgetragene Partikel jedoch grösser als rote Blutzellen sind, ist das Risiko gross, dass sie die Blutbahn an anderer Stelle verstopfen und somit zu einer Embolie führen können.

Ferner ist es aus der Literatur bekannt, die abgetragenen Partikel durch den Katheter abzusaugen. Dabei besteht jedoch das Risiko, dass zu viele Partikel nicht erfasst werden, so dass diese in die Blutbahn gelangen.

Der Erfindung liegt deshalb die Aufgabe zugrunde, einen Katheter der eingangs genannten Art zu schaffen, bei dem die abgetragenen Partikel nahezu vollständig aus dem Blutkreislauf entfernt werden.

Die gestellte Aufgabe wird erfindungsgemäss durch die im Anspruch 1 angegebenen Merkmale gelöst.

Der erfindungsgemässe Katheter besitzt eine Förderschraube mit einem erheblich verbesserten Wirkungsgrad und gewährleistet daher einen sofortigen und stetigen Wegtransport der abgetragenen bzw. abgelösten Partikel, um mit Sicherheit zu vermeiden, dass diese in den Blutkreislauf gelangen.

Eine Ausführungsform nach Anspruch 2 stellt konstruktiv eine besonders einfache Lösung dar.

Durch eine Ausführungsform nach Anspruch 3 lässt sich der Wirkungsgrad noch weiter steigern, da die Fläche eines Vierkantquerschnitts eine höhere Förderleistung erbringt als beispielsweise ein runder Drahtquerschnitt.

Eine Ausführungsform nach Anspruch 4 ermöglicht einerseits die freie Auswahl eines bezüglich Festigkeit besonders geeigneten Werkstoffes und andererseits einen geeigneten Schutz vor Korrosion sowie die Erfüllung entsprechender Forderungen in hygienischer und tribologischer Hinsicht.

Anspruch 5 beschreibt eine besonders bevorzugte Ausführungsform, bei der die Partikel unmittelbar nach dem Ablösen durch das Fenster auf die Förderschraube gelangen. Dadurch wird weitgehend vermieden, dass abgelöste Partikel in die Blutbahn gelangen können.

Bei einer Ausführungsform nach Anspruch 6 wird im Gegensatz zu frei schneidenden Messern durch die Scherwirkung ein besser kontrollierbares Abtrennen der Ablagerungen erzielt und dabei auch die Gefahr von Verletzungen der Blutgefässwandungen reduziert. Insbesondere ist durch eine solche Ausführungsform auch besser kontrollierbar, dass die abgetrennten Partikel mit grosser Wahrscheinlichkeit durch das oder die Fenster in den Bereich der Förderschraube gelangen und somit vom Eintritt in die Blutbahn ferngehalten werden.

Bei einer bevorzugten Ausführungsform nach Anspruch 7 greift der Rotor radial an den Ablagerungen an. Dadurch wird verhindert, dass beispielsweise im Bereich von Krümmungen geradeaus in die Gefässwand gebohrt werden kann.

Die weiteren abhängigen Ansprüche kennzeichnen weitere bevorzugte Ausführungsformen der Erfindung.

Anhand der Zeichnungen wird ein Ausführungsbeispiel der Erfindung näher erläutert. Es zeigen:
- Fig. 1: Einen Rotationskatheter in Gesamtansicht, mit Antrieb, Führungsdraht und Auffangbehälter für die aus dem Blutgefäss abgelösten Ablagerungsteile,
- Fig. 2: den Kopfteil des Rotationskatheters nach Fig. 1, jedoch in einem grösseren Massstab, im Grundriss,
- Fig. 3: einen Längsschnitt durch den Kopfteil des Rotationskatheters gemäss Fig. 2 und
- Fig. 4: Führungsdraht und Förderschraube in einem noch grösseren Massstab.

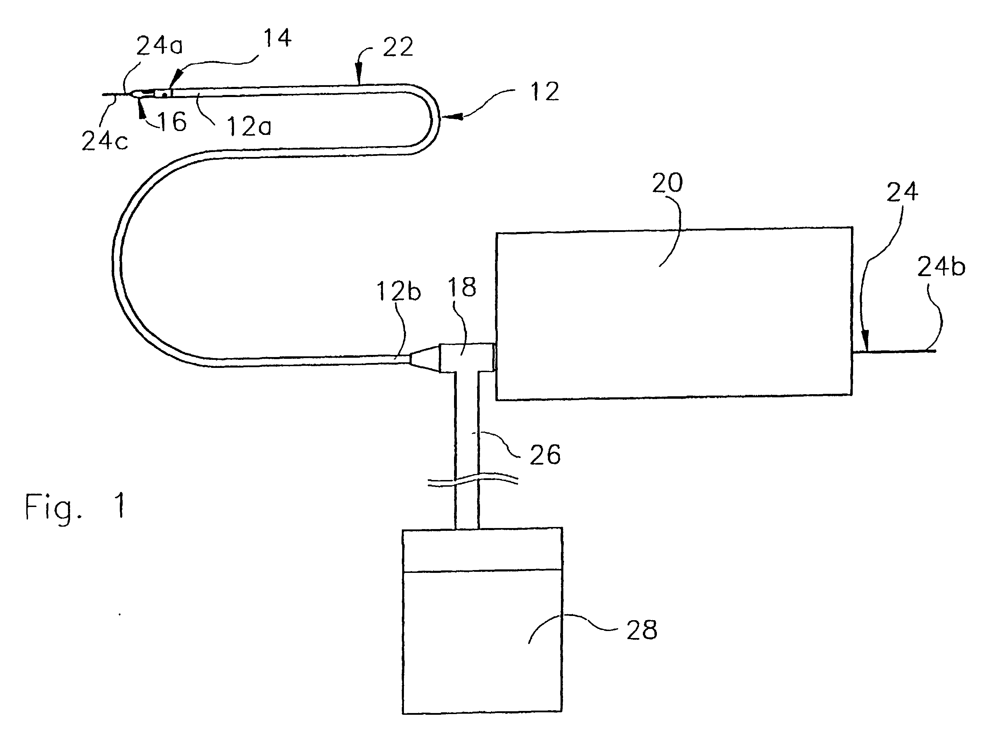

Der in Fig. 1 dargestellte Katheter **12,** weist an seinem vorderen Ende **12a** ein aus Stator **14** und Rotor **16** bestehendes Schneidwerkzeug auf. An seinem hinteren Ende **12b** ist der Katheter **12** über eine Abfuhrkammer **18** mit einem Drehantrieb **20** verbunden. In einer als Katheterschlauch dienenden schlauchförmigen Hülle **22** ist eine flexible Antriebswelle gelagert, die den Rotor **16** mit dem Drehantrieb **20** verbindet. Durch die gesamte Länge des Katheters **12** hindurch erstreckt sich ein Führungsdraht **24**, dessen vorderes Ende **24a** aus dem Rotor **16** und dessen hinteres Ende **24b** aus dem Drehantrieb **20** herausragt. Der Führungsdraht **24** weist an seinem vorderen Ende **24a** eine Federspitze **24c** auf. An die Abfuhrkammer **18** ist in radialer Richtung über einen Schlauch oder ein Rohr **26** ein Auffangbehälter **28** angeschlossen.

Bei der Anwendung des Katheters **12** wird der Führungsdraht **24** mit seinem vorderen Ende **24a** voraus in die zu behandelnde Arterie oder Vene eingeführt und anschliessend unter Röntgenkontrolle bis an die verengte Stelle und durch diese hindurch manövriert. Hierauf wird der Katheter 12 auf dem Führungsdraht **24** nachgeführt. Sobald das vordere Ende **12a** die zu behandelnde Stelle erreicht hat, wird der Drehantrieb **20** eingeschaltet, um die unerwünschten Ablagerungen mittels des Schneidwerkzeuges **14, 16** abzulösen und aus der Blutbahn herauszufördern. Die Drehzahl des Rotors **16** liegt vorzugsweise im Bereich zwischen 30'000 und 40'000/min. Während des Betriebes wird der Katheter **12** langsam vorgeschoben. Die abgetrennten und zerkleinerten Ablagerungen werden durch die schlauchförmige Hülle **22** bis zur Abfuhrkammer **18** weggefördert und gelangen von dort in den Auffangbehälter **28**.

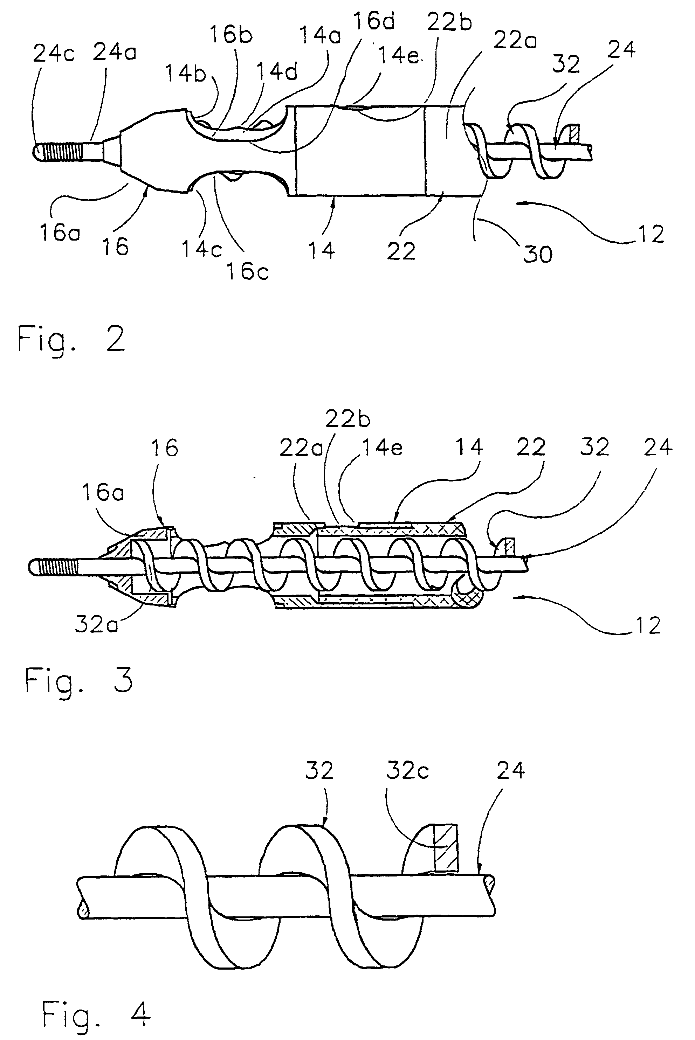

Die Figuren 2 und 3 zeigen das vordere Ende **12a** des Katheters **12** mit seinem Stator **14**, seinem als Aussenläufer ausgebildetem Rotor **16,** seiner schlauchförmigen Hülle **22** sowie dem vorderen Ende **24a** des Führungsdrahtes **24**. Die durch den Schnitt **30** unterbrochen dargestellte schlauchförmige Hülle **22** lässt die flexible Antriebswelle **32** erkennen, deren vorderes Ende **32a** mit dem Rotor **16** drehund zugfest verbunden ist. Innerhalb der Antriebswelle **32** erstreckt sich der Führungsdraht **24.** Die Antriebswelle **32** ist als Förderschraube ausgebildet, um die durch das Schneidwerkzeug **14, 16** abgetragenen Ablagerungen durch die schlauchförmige Hülle **22** zur Abfuhrkammer **18** (Fig. 1) zu fördern.

Ein Abschnitt **14a** des Stators **14** erstreckt sich in den Rotor **16** hinein. Es ist ersichtlich, dass der Statorabschnitt **14a** und der Rotor **16** hülsenförmig ineinandergreifen. Der Statorabschnitt **14a** weist zwei umfangsseitig um 180° zueinander versetzte Scherfenster **14b, 14c** auf. Der Rotor **16** weist ebenfalls zwei umfangsseitig um 180° zueinander versetzte Scherfenster **16b, 16c** auf.

Das Fenster **14b** des Statorabschnitts **14a** ist in Umfangsrichtung schmäler als dasjenige **16b** des Rotors **16**. Die eine Kante des Rotorfensters **16b** ist als Schneide **16d** ausgebildet. Die entgegengerichtete Kante des Statorfensters **14b** ist als Gegenschneide **14d** ausgebildet. Diese Gegenschneide **14d** verläuft in axialer Richtung mindestens annähernd wellenförmig, bezogen auf eine zylindrische Fläche.

Die Schneide **16d** und die Gegenschneide **14d** wirken scherenartig zusammen. Derartige Schneiden sind jeweils in beiden, auch als Scherfenster zu bezeichnenden Fenstern **14c, 16c,** umfangsseitig um 180° zueinander versetzt angeordnet.

Der Rotor **16** weist in Richtung seiner Spitze ein im Durchmesser abnehmendes vorderes Ende **16a** auf. Dadurch wird die verengte und zu behandelnde Stelle der Arterie oder Vene beim Einführen des Katheters **12** aufgeweitet.

Rotor **16** und Stator **14** bestehen vorzugsweise aus Metall. Der Führungsdraht **24** mit der Federspitze **24c** ist ein Stahldraht. Die als Förderschraube dienende Antriebswelle **32** besteht aus einem beispielsweise beschichteten Stahldraht. Die schlauchförmige Hülle **22** besteht aus Kunststoff.

Zur drehfesten Verbindung des Stators **14** mit der schlauchförmigen Hülle **22** wird deren Ende **22a** in den Stator **14** eingepresst. Zur Fixierung sind in der Mantelfläche des Stators **14** Löcher **14e** angeordnet, in welche das eingepresste Schlauchmaterial **22b** formschlüssig hineinquillt.

Die Fig. 3 zeigt insbesondere, dass sich die Antriebswelle **32** bis in das vordere Ende **16a** des Rotors **16** hineinerstreckt und deren vorderes Ende **32a** mit dem Rotor **16** dreh- und zugfest verbunden, beispielsweise in diesen eingepresst ist. Ebenfalls ersichtlich ist, wie die - schlauchförmige Hülle **22** im Stator **14** durch die Löcher **14e** formschlüssig gesichert ist.

Die Fig. 4 zeigt den rechteckigen Querschnitt des Drahtes **32c** der als Förderschraube dienenden, wendelförmigen Antriebswelle **32.** Durch die Anordnung des Führungsdrahtes **24** koaxial innerhalb der Antriebswelle **32** ergibt sich ein besonders hoher Wirkungsgrad als Förderschraube. Die Förderung der abgetragenen Teile der Ablagerungen erfolgt innerhalb des Katheterschlauches **22** nahezu linear.

## Patentansprüche

1. Katheter zum Ablösen von abnormalen Ablagerungen in menschlichen Blutgefässen, wie Arterien oder Venen, mit einem an seinem vorderen Ende (**12a**) angeordneten, einen Rotor (**16**) aufweisenden Schneidwerkzeug (**14, 16**)**,** welches über eine in einer schlauchförmigen Hülle (**22**) angeordneten flexiblen Antriebswelle (**32**) mit einem am hinteren Ende (**12b**) des Katheters (**12**) anschliessbaren Drehantrieb (**20**) verbindbar ist, wobei die flexible Antriebswelle als Förderschraube (**32**) ausgebildet und derart gewendelt ist, dass sie in angetriebenem Zustand die abgetrennten Ablagerungen in Richtung des Drehantriebs (**20**) fördert, **dadurch gekennzeichnet, dass** sich ein vom Katheter unabhängig bewegbarer Führungsdraht (**24**) koaxial durch die als Förderschraube ausgebildete flexible Antriebswelle (**32**) hindurcherstreckt.

2. Katheter nach Anspruch 1, **dadurch gekennzeichnet, dass** die als Förderschraube ausgebildete Antriebswelle (**32**) aus einem gewendelten Draht (**32c**) besteht.

3. Katheter nach Anspruch 2, **dadurch gekennzeichnet, dass** der Draht (**32c**) im Querschnitt im wesentlichen rechteckig ist.

4. Katheter nach Anspruch 3, **dadurch gekennzeichnet, dass** der Draht (**32c**) beschichtet ist

5. Katheter nach einem der vorstehenden Ansprüche, **dadurch gekennzeichnet, dass** das Schneidwerkzeug (**14**, **16**) mindestens ein Fenster (**14b, 14c, 16b, 16c**) aufweist, durch welches die Ablagerungen beim Abtrennen ins Innere des Schneidwerkzeuges (**14, 16**) gefördert werden und dass sich die Antriebswelle (**32**) bis in das Innere des Schneidwerkzeuges (**14, 16**) hineinerstreckt.

6. Katheter nach Anspruch 5, **dadurch gekennzeichnet, dass** das Schneidwerkzeug (**14, 16**) einen Stator (**14**) mit einem Statorabschnitt (**14a**) aufweist und dass am Statorabschnitt (**14a**) und am Rotor (**16**) scherenartig zusammenwirkende Schneiden (**14d, 16d**) angeordnet sind.

7. Katheter nach Anspruch 6, **dadurch gekennzeichnet, dass** der Statorabschnitt (**14a**) und der Rotor (**16**) wenigstens im Bereich der Schneiden (**14d**, **16d**) zumindest annähernd zylinderförmig ausgebildet sind, dass der Rotor (**16**) den Statorabschnitt (**14a**) als Aussenläufer umgibt und dass die Schneiden (**14d**, **16d**) in den Mantelflächen des Rotors (**16**) und des Statorabschnitts (**14a**) angeordnet sind.

8. Katheter nach Anspruch 7, **dadurch gekennzeichnet, dass** die Fenster (**14b**, **14c**, **16b**, **16c**) in den Mantelflächen des Statorabschnitts (**14a**) und des Rotors (**16**) angeordnete Scherfenster sind, deren Kanten als die Schneiden (**14d, 16d**) ausgebildet sind.

9. Katheter nach Anspruch 8, **dadurch gekennzeichnet, dass** im statorabschnitt (**14a**) und/oder im Rotor (**16**) gleichmässig über den Umfang verteilt mindestens zwei Scherfenster (**14b**, **14c, 16b, 16c**) angeordnet sind.

## Claims

1. Catheter for detaching abnormal deposits from blood vessels in humans, such as arteries or veins, with a cutting tool (14, 16) which is arranged at its front end (12a), has a rotor (16) and can be connected, via a flexible drive shaft (32) arranged in a tubular sheath (22), to a rotary drive mechanism (20) which can be attached at a rear end (12b) of the catheter (12), the flexible drive shaft being designed as a conveyor screw (32) and being wound helically in such a way that when it is being driven, it conveys the dislodged deposits in the direction of the rotary drive mechanism (20), **characterized in that** a guide wire (24) which can be moved independently of the catheter extends coaxially right through the flexible drive shaft (32)designed as a conveyor screw.

2. Catheter according to Claim 1, **characterized in that** the drive shaft (32) designed as a conveyor screw consists of a helically wound wire (32c).

3. Catheter according to Claim 2, **characterized in that** the wire (32c) is substantially rectangular in cross-section.

4. Catheter according to Claim 3, **characterized in that** the wire (32c) is coated.

5. Catheter according to one of the preceding claims, **characterized in that** the cutting tool (14, 16) has at least one slot (14b, 14c, 16b, 16c) through which the deposits are conveyed into the inside of the cutting tool (14, 16) when dislodged, and **in that** the drive shaft (32) extends into the inside of the cutting tool (14, 16).

6. Catheter according to Claim 5, **characterized in that** the cutting tool (14, 16) has a stator (14) with a stator portion (14a), and **in that** cutting edges (14d, 16d) are arranged on the stator portion (14a) and on the rotor (16) to interact in a shearing action.

7. Catheter according to Claim 6, **characterized in that** the stator portion (14a) and the rotor (16) are designed at least approximately cylindrical, at least in the region of the cutting edges (14d, 16d), **in that** the rotor (16) surrounds the stator portion (14a) as external rotor, and **in that** the cutting edges (14d, 16d) are arranged in the circumferential surfaces of the rotor (16) and of the stator portion (14a).

8. Catheter according to Claim 7, **characterized in that** the slots (14b, 14c, 16b, 16c) are shearing slots which are arranged in the circumferential surfaces of the stator portion (14a) and of the rotor (16) and whose margins are designed as the cutting edges (14d, 16d).

9. Catheter according to Claim 8, **characterized in that** at least two shearing slots (14b, 14c, 16b, 16c) are arranged, uniformly distributed about the circumference, in the stator portion (14a) and/or in the rotor (16).

## Revendications

1. Cathéter pour détacher des dépôts anormaux dans des vaisseaux sanguins humains tels que des artères ou des veines, comportant un outil de coupe (14, 16) présentant un rotor (16) et agencé à son extrémité antérieure (12a), qui peut être relié via un arbre moteur (32) flexible agencé dans un manchon tubulaire (22) avec un entraînement rotatif (20) qui peut être raccordé à l'extrémité postérieure (12b) du cathéter (12), l'arbre moteur étant réalisé sous forme de vis sans fin (32) qui est spiralée de telle sorte qu'à l'état entraîné, elle transporte les dépôts détachés en direction de l'entraînement rotatif (20), **caractérisé en ce qu'**un fil de guidage (24) se déplaçant indépendamment du cathéter s'étend coaxialement à travers l'arbre d'entraînement (32) flexible réalisé sous forme de vis sans fin.

2. Cathéter selon la revendication 1, **caractérisé en ce que** l'arbre d'entraînement (32) réalisé sous forme de vis sans fin est constitué par un fil spiralé (32a).

3. Cathéter selon la revendication 2, **caractérisé en ce que** le fil (32c) a une section transversale sensiblement rectangulaire.

4. Cathéter selon la revendication 3, **caractérisé en ce que** le fil (32c) est revêtu.

5. Cathéter selon l'une quelconque des revendications précédentes, **caractérisé en ce que** l'outil de coupe (14, 16) présente au moins une fenêtre (14b, 14c, 16b, 16c) à travers laquelle lors de leur séparation, les dépôts sont transportés jusqu'à l'intérieur de l'outil de coupe (14, 16) et **en ce que** l'arbre moteur (32) s'étend jusqu'à l'intérieur de l'outil de coupe (14, 16).

6. Cathéter selon la revendication 5, **caractérisé en ce que** l'outil de coupe (14, 16) présente un stator (14) avec un tronçon de stator (14a) et **en ce que** des tranchants (14d, 16d) qui coopèrent à la manière de ciseaux sont agencés sur le tronçon de stator (14a) et sur le rotor (16).

7. Cathéter selon la revendication 6, **caractérisé en ce que** le tronçon de stator (14a) et le rotor (16) sont réalisés au moins dans la région des tranchants (14d, 16d) au moins de manière approximativement cylindrique, **en ce que** le rotor (16) entoure le tronçon de stator (14a), en tant qu'induit extérieur, et **en ce que** les tranchants (14d, 16d) sont agencés dans les surfaces enveloppes du rotor (16) et du tronçon de stator (14a).

8. Cathéter selon la revendication 7, **caractérisé en ce que** les fenêtres (14b, 14c, 16b, 16c) sont des fenêtres de cisaillement agencées dans les surfaces enveloppes du tronçon de stator (14a) et du rotor (16), dont les arêtes sont réalisées à titre des tranchants (14d, 16d).

9. Cathéter selon la revendication 8, **caractérisé en ce que** dans le tronçon de stator (14a) et/ou dans le rotor (16) sont agencées au moins deux fenêtres de cisaillement (14b, 14c, 16b, 16c) qui sont réparties de façon régulière sur la périphérie.
